# EUROPEAN PATENT APPLICATION

(11) **EP 1 623 707 A1**
(43) Date of publication of application: **08.02.2006**
(21) Application number: 03774131.1
(22) Date of filing: 21.11.2003
(51) Int. Cl.: A61K 31/352, A61P 27/16, A61P 43/00, C07D 311/36

(54) **MEDICINAL COMPOSITION FOR TREATING SUDDEN DEAFNESS**

(30) Priority: 21.11.2002 JP 2002338026
(71) Applicant: Nichimo Co., Ltd, Tokyo 140-0002 (JP)
(72) Inventor: TAKEBE, Minoru, Shinagawa-ku, Tokyo 140-0002 (JP); PAN, Weijun, Shinagawa-ku, Tokyo 140-0002 (JP)
(74) Representative: Main, Malcolm Charles
(86) International application number: PCT/JP2003/014891
(87) International publication number: WO 2004/045603

(57) **Abstract**

The pharmaceutical composition of the present invention for treating sudden deafness containing, as an active ingredient, isoflavone aglycone derived from soybean germ can be used to prevent sudden deafness in humans by manifesting an action that is effective in treating disease in humans. The pharmaceutical composition for treating sudden deafness of the present invention is **characterized in that** it contains a product that is an isoflavone aglycone concentrate and is obtained by fermenting soybean germ, that is a raw material, using koji mold thus decomposing proteins; then executing hydrolysis thereon; and extracting and concentrating a resulting product using a solvent.

## Description

### Technical Field

The present invention pertains to a pharmaceutical composition for treating sudden deafness containing isoflavone aglycone derived from soybean germ, or another isoflavone aglycone, having an action that is effective in treating sudden deafness in humans.

### Background Art

The applicant of the present application previously proposed in Japanese Patent 3,014,145 a method for producing isoflavone aglycone derived from soybeans, suggesting that the product obtained by fermenting a legume raw material using koji mold to decompose the protein and then performing hydrolysis is rich in isoflavone aglycone because soybean isoflavone glycoside is converted to isoflavone aglycone by an enzyme of koji mold (β-glycosidase), and that this product acts to promote drug effects and other biological activities in humans and mammals.

It has been reported in recent years that isoflavone compounds have these drug effects and other biological activities. For example, it is known that isoflavone compounds have estrogen activity, antiestrogen activity, antioxidant activity, antihemolysis activity, antibacterial activity, antilipemia activity, anticholesterol activity, spasmolytic activity, the ability to induce differentiation of cancer cells, cancer gene-inhibiting activity, anticancer effects, the ability to prevent arteriosclerosis, immune-activating activity, the ability to enhance hemopoietic stem cells, and other effects. Of the isoflavone compounds, isoflavone aglycone has a particularly high by body absorption rate; therefore, there are many reports relating to the functionality thereof. However, thus far there have been no reports on the function of isoflavone aglycone for the treatment of sudden hearing loss.

In 1973, the Ministry of Health and Welfare in Japan launched a research group for sudden deafness as an intractable disease and announced the guidelines for diagnosing sudden hearing loss as described below:

### <Primary Symptoms>

1. Sudden hearing loss (as the name implies, instantaneous hearing loss, or hearing loss that the patient notices when he/she wakes up in the morning)
2. Severe sensorineural hearing loss (the actual problem is not that it is necessarily "severe," but that unless it is "severe," sudden hearing loss often goes unnoticed)
3. Etiology is unknown or uncertain (that is, the etiology is unclear)

### <Secondary Symptoms>

1. Tinnitus (tinnitus develops at approximately the same time as hearing loss occurs)
2. Dizziness and/or Vertigo, nausea, vomiting (vertigo and nausea and vomiting approximately accompany hearing loss, but there are not recurring attacks of vertigo)

### <Diagnostic criteria>

1. Definite cases of sudden hearing loss will display all of the primary symptoms and the secondary symptoms.
2. Suspected cases of sudden hearing loss will display the primary symptom 1 and 2.

The name sudden hearing loss also in a broad sense implies all sensorineural hearing loss that occurs suddenly, but the name "sudden deafness" or "acute sensorineural hearing loss" is generally used in this case and is limited to hearing loss of unknown or uncertain etiology. That is, trauma, drug-induced ototoxicity, complication of multiple maladies, iatrogenic hearing loss, and Meniere's syndrome are among the forms of hearing loss that are differentiated from sudden deafness.

In addition, disturbance of inner ear circulation, viral infection, endolymphatic edema, rupture of the oval window or the round window, allergy, and the like are cited as causes of sudden deafness. However, there are still many unknown points concerning sudden deafness and sudden deafness does not have a unitary etiology. The above-described causes of sudden deafness also induce disturbance of circulation. The pathology of disturbance of inner ear circulation today suggests infarct, hemorrhage, thrombus, and the like.

The following are methods for treating sudden deafness.
1. Improvement of inner ear circulation
   a) Treatments that directly affect the blood vessels
      Nicotinic acid agents, circulatory hormones, Meylon (aqueous 7% sodium bicarbonate), carbon dioxide inhalation therapy, papaverine agents
   b) Treatments that promise autonomic nerve activity
      Stellate block, histamines, pilocarpine therapy
2. Brain metabolism activation and antineuritic therapy
   B vitamins, nicotine, Embol, Lucidril, solcoseril, cytochrome C, ATP, CoQ10 (coenzyme Q10)
3. Remission of coagulation disorders
   Low-molecular-weight dextran, heparin
4. Antiviral therapy
   γ-Globulin, antibiotics, interferon
5. Treatment for hydrolabyrinth
   Diuretics, meylon (aqueous 7% sodium bicarbonate), merislon
6. Surgical therapy
   Surgery to close a ruptured labyrinthine wall
7. Recent therapies
   Hyperbaric oxygen therapy, amidotrizoate, ultra-short wave therapy

Isoflavone aglycone derived from soybeans has never been studied for the purpose of treatment by any of these therapeutic methods.

It is estimated that the number of patients treated for sudden deafness throughout Japan reached 14,000 to 19,000 (average of approximately 16,700 people) per year in 1987, which was three-times to four-times the number in 1972, and a further increase to 21,000 to 27,000 people (average of approximately 24,000 people) per year was seen in 1993. The survey conducted in 1993 showed that the male to female ratio among patients with sudden deafness was 0.94:1.00, but the results of other surveys indicate that there is not a difference between the sexes. In contrast to the fact that the incidence of sudden deafness increases in men ranging in age from their forties to their seventies, the incidence of sudden deafness increases greatly overall in women ranging in age from their late twenties to their seventies. There is particularly an increase in women ranging in age from their fifties to their seventies; therefore, changes in environmental or social factors can be presumed. Although sudden deafness is not a life-threatening disease, sudden "hearing loss" and "tinnitus" can be a serious problem to a healthy person with an active lifestyle when it is occasionally accompanied by severe vertigo. There is also the possibility that women's participation in public affairs and increased stress have an affect on the occurrence of sudden hearing loss. There will probably be an increase in the number of people who develop sudden hearing loss in the future unless today's public environment improves. Furthermore, the number of people who develop sudden deafness is similarly increasing in other developed countries.

Thus, there is a need for a substance capable of manifesting drug effects and the like that are superior for the treatment of the serious problem of sudden deafness.

### Disclosure of Invention

The present invention was made in light of the points described above, and the object of the present invention is to provide a pharmaceutical composition for treating sudden deafness that contains isoflavone aglycone derived from soybean germ or other isoflavone aglycone having an action that is effective for treating sudden deafness in humans.

### [Means to solve problems]

The inventor of the present application, as a result of intense researches, produced a product proposed previously in Japanese Patent No. 3014145, which is a "product that is obtained with the use of soybean germ as raw material bean, wherein koji preparation is performed on the soybean germ using koji mold, and its resultant is subjected to a hydrolysis treatment, thus obtaining a product that contains a large amount of isoflavone aglycones or a product in which the phytic acid has been completely removed by decomposition"; and on this product, in accordance with the production method proposed in Japanese Patent Application Laid-Open (Kokai) No. 2000-281673 filed by the inventor of the present application and other inventor(s), with a use of solvent, extraction and concentration was performed, thus obtaining a product which is a concentrate with an isoflavone aglycone level of 30 wt% or greater (to which a mark "AglyMax" (Japanese registered trademark of Nichimo Co., Ltd., thus the product being referred to as "AglyMax" below) was applied); and further, this AglyMax was administered subjects suffering from sudden deafness, and it was confirmed that sudden hearing loss was alleviated. The present invention was thus completed based upon the confirmation. The isoflavone aglycone is not limited to that contained in soybean germ and includes isoflavone aglycone contained in parts of the soybean other than the germ, other legumes, and other plants, as well as isoflavone aglycone produced by chemical synthesis, and other means.

As a result of further intense research, the inventors perfected the present invention upon clarifying that AglyMax has very strong superoxide scavenging activity and this superoxide scavenging activity alleviates sudden hearing loss.

This superoxide scavenging activity will be described in further detail below. Superoxides (O₂') are a type of active oxygen that are produced by neutrophils and macrophages and protect the body from impurities, but also are the cause of pulmonary emphysema, cataracts, arteriosclerosis, psoriasis, and the like. These superoxides are, therefore, a double-edged sword. The enzyme activity of superoxide dismutase (SOD) and superoxide scavenging activity (SOSA or SOD-like activity) are examples of the ability of food products to eliminate O₂'. The former is an enzyme and is therefore broken down by digestive enzymes and goes virtually unabsorbed in vivo when taken orally. Consequently, the latter is promising for food products. AglyMax has the latter SOD-like activity. ESR is a method for measuring SOD-like activity and assessing the ability of AglyMax to scavenge O₂'. By means of this method, O₂' generation is assessed by using a hypoxanthine-xanthine oxidase system and DMPO (5,5-dimethyl-1-pyrroline-N-oxide) as the spin trap agent and measuring DMPO-OOH (mixed with DMPO-OH) as the spin adduct. This method is also called the spin trap method.

In accordance with preparation by this spin trap method, only the water-soluble components are extracted in order to extract the component with SOD-like activity contained in a sample with 0.1 M phosphate buffer. Consequently, when AglyMax (AglyMax-70; 70% isoflavone aglycone concentrate) was extracted and prepared with untreated 0.1 M phosphate buffer and used for superoxide scavenging activity, superoxide scavenging activity was 12,000 units/g. Nevertheless, the water-soluble component of AglyMax is removed during the process whereby isoflavone aglycone is extracted and concentrated from fermented soybean germ; therefore, the inventors and others noted that because this isoflavone will not dissolve in 0.1 M phosphate.buffer, the superoxide scavenging activity thereof cannot be accurately measured. Consequently, measuring superoxide scanvenging activity with the isoflavone pre-emulsified in a small amount of oil or fat was contemplated. It became evident that when an emulsifier (Tween-80 or other emulsifier) was added to emulsify AglyMax and the superoxide scavenging activity was measured, the superoxide scanvenging activity of AglyMax was extremely high at 160,000 units/g.

When the isoflavone aglycone reagents of daidzein and genistein were analyzed for comparison with the SOD-like activity of AglyMax, their SOD-like activity was extremely low at 300 units/g or less, regardless of whether or not they were emulsified (the results are shown in Table 1). Therefore, it can be assumed that the high SOD-like activity of AglyMax obtained by fermentation of soybean germ using koji mold and concentration of the resulting isoflavone aglycone is not due to isoflavone aglycone, but instead to a component that is produced by fermentation. The SOD-like activity of the product of company F (brand name: "Fujiflavone", Fujicco Co., Ltd.) that is obtained by concentrating and extracting isoflavone glycoside from soybean germ without fermenting the soybean germ was analyzed, but was 300 units/g or less. That is, it became clear that a component high in SOD-like activity is not present in soybean germ that has not been subjected to fermentation by koji mold. The SOD-like activity of the product of company K (brand name: "SoyAct", Kikkoman Corporation) that is obtained by fermentation of whole soybeans using koji mold to produce isoflavone aglycone and concentrating and extracting this isoflavone aglycone was measured, but it was also low at 300 units/g or less. Therefore, it can be said that a component that is very high in SOD-like activity is present in soybean germ as a result of fermentation.

**Table 1 Comparison of the SOD-like Activity of AglyMax and Other Products**

| | Genistein | Daidzein | Company F preparation | Company K preparation | Product of present invention |
|---|---|---|---|---|---|
| SOD-like | 300 or less | 300 or less | 300 or less | 300 or less | 12,000 |
| activity | (300 or less) | (300 or less) | (300 or less) | (300 or less) | (160,000) |

| | | | | | |
|---|---|---|---|---|---|
| Units: units/g | | | | | |
| The figures in parentheses are the activity after emulsification. | | | | | |

On the other hand, the superoxide scavenging activity (SOD-like activity) of food products is virtually from water-soluble component, and polyphenols, which are water-soluble, have a high SOD-like activity. The SOD-activity of grapeseed extract, ginkgo leaf extract and guava leaf extract is high at 100,000 to 200,000 units/g, and this activity is said to be from polyphenols.

As described above, it is confirmed that AglyMax has SOD-like activity on the same level as these food products, but because the component contained in AglyMax with a high SOD-like activity is not water-soluble but rather is close to being fat-soluble, it is promising as a substance that has an effect or function that is different from that of grapeseed extract, ginkgo leaf extract, or guava leaf extract.

It is understood that superoxides (O₂') do not damage human bodies extracellularly; instead, superoxides damage the genes and the like inside the cells to become the cause of disease. In other words, although it is known that a fat-soluble component and not a water-soluble component can pass through the cell membrane covered with a cytoplasm layer and have superoxide scavenging activity inside the cell, and there was nothing resembling this fat-soluble component among natural components until now.

Now, it is completely within the realm of possibility that sudden deafness, which is identified as a malady that is very difficult to treat, can be treated using the high SOD-like activity characteristic of the fat-soluble component of AglyMax, which has not been known among natural components until now.

The pharmaceutical composition for treating sudden hearing loss of the present invention obtained in this way is characterized in that it contains isoflavone aglycone as an active ingredient.

In addition, the pharmaceutical composition for treating sudden hearing loss containing isoflavone aglycone derived from soybean germ of the present invention is characterized in that it contains as the active ingredient a product obtained by fermenting soybean germ raw material using koji mold in order to decompose proteins; then performing hydrolysis, and further performing extraction and concentration on the resulting product using a solvent.

In the pharmaceutical composition of the present invention the composition of the isoflavone aglycone in the concentrate is at least 70 wt% of daidzein.

Furthermore, the isoflavone aglycone concentrate is characterized in that it has high superoxide scavenging activity.

The pharmaceutical composition for treating sudden deafness containing as isoflavone aglycone as an active ingredient and the pharmaceutical composition for treating sudden deafness containing isoflavone aglycone derived from soybean germ of the present invention made in this way manifest an action that is effective in treating sudden hearing loss in humans and can therefore be used to treat sudden deafness of various etiologies.

More specifically, this isoflavone aglycone promises to be effective in improving the disturbance of inner ear circulation that is the primary cause of sudden deafness, as noted below. Furthermore, there is a strong possibility that it will also help to improve female hormone imbalance, activate the immune system, and alleviate stress.
1) AglyMax enhances the ability of erythrocytes to deform and thereby functions to improve cochlear blood flow of the inner ear.
2) AglyMax activates the NO (nitrogen monoxide)-synthesizing enzymes (eNOS) in the vascular endothelial cells, relaxes the cochlear arteries of the inner ear, and improves blood flow.
3) In females, AglyMax prevents female hormone (estrogen) imbalance and improves dysfunction of the autonomic nervous system caused by hormonal imbalance (anti-estrogen activity can be expected when estrogen secretion is high, while it has estrogen-like activity when there is no estrogen secretion after menopause).
4) AglyMax activates the immune system and alleviates stress.

Although the pharmaceutical composition for treating sudden deafness of the present invention promises to be effective in the treatment of sudden deafness when isoflavone aglycone only is used, it can also be made into a pharmaceutical preparation in combination with B vitamins, nicotine, Embol, Lucidril, solcoseril, cytochrome C, ATP, CoQ10 (coenzyme Q10), and other substances that are promising as brain metabolism activation and antineuritic therapy.

Because the pharmaceutical composition for treating sudden deafness containing isoflavone aglycone derived from soybean germ as an active ingredient of the present invention is made and acts as previously described, it can be used to prevent disease in humans by manifesting an action that is effective in treating sudden deafness in humans.

### Brief Description of Drawings

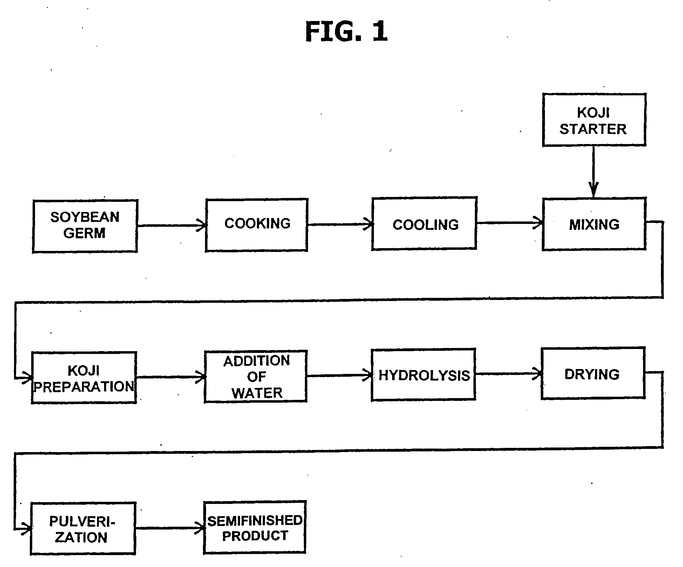

Figure 1 is a block diagram showing an example of the method for producing the aglycone that has strong pharmaceutical activity of isoflavone compounds from soybean germ of the present invention.

### Best Mode for Carrying Out the Invention

Embodiments of the present invention will now be described below.

In this embodiment, a product previously proposed by the inventor of the present application in Japanese Patent No. 3,014,145 is employed in which the "product is one obtained by using soybean germ as raw material bean (pulse crop), wherein koji preparation is performed on the soybean germ using koji mold, and its resultant is subjected to a hydrolysis treatment, so that the obtained product is a product which contains a large amount of isoflavone aglycones or is a product in which the phytic acid has been completely removed by decomposition"; and on the thus obtained product(s), in accordance with the production method disclosed in Japanese Patent Application Laid-Open (Kokai) No. 2000-28167 filed by the inventor of the present application and other inventor(s), solvent is used for extraction and concentration to produce a concentrate product that contains isoflavone aglycone at, for example, 30 wt% or greater.

Figure 1 shows the product proposed in Japanese Patent No. 3,014,145 referred to above, and it is a process diagram that presents one embodiment of the product manufacture method wherein glycosides contained in the isoflavone compounds from the germ of soybean, which is a type of bean (pulse crop), are decomposed to generate isoflavone compounds rich in aglycone. Figure 1 also shows one embodiment for a method for manufacturing product produced by the removal of phytic acid in soybean meal.

First, a description will be presented regarding the manufacture of product that generates isoflavone compounds that are rich in aglycone.

In accordance with the process of Figure 1, soybean germ is first cooked, thereby facilitating the propagation of koji mold. The soybean germ can be cooked in a batch format or continuous format in accordance with factors such as manufacture objectives.

The soybean germ that has been cooked is then cooled, and the water content in the soybean germ is adjusted to an amount that allows the propagation of the koji mold (e.g., 37 wt%).

Using the soybean germ with the water content adjusted in this manner, the method of the present invention is then carried out in the manner described below.

More specifically, the cooked soybean germ is sterilized, and after cooling, the blending ratio of soybean germ and koji mold is set by mixing 400 kg of soybean germ and 200 g of koji starter with a koji mold count adjusted (using polished rice) to 8 x 10⁷/g. In addition, after cooling the mixture to 32°C at the start of koji preparation, the temperature of the product is allowed to increase to 40°C without providing ventilation, and temperature increase is then controlled by ventilating at the point when 40°C is reached. A first stirring is initiated at about 17 hours from the start ("*mori*" (ramp-up) process). The soybean germ is cooled, and the temperature is controlled while ventilating so that the product temperature is about 35°C after stirring. Next, a second stirring process is performed after about eight hours ("naka"(intermediate) process), thus cooling the product. Again, the product temperature is controlled by ventilation at about 35°C; and after about 16 hours, a third stirring process is carried out in the same manner as above *("shimai"* (finishing) process). While subsequently ventilating so that the product temperature is controlled at about 38°C, koji preparation is completed at about 48 hours from the start. Upon completion of koji preparation, the water content is adjusted to 50% while stirring, and the product is heated so that the product temperature reaches about 50°C. Hydrolysis is performed for 48 hours or longer by the enzyme in the koji mold so that most of the isoflavone compounds in the soybean germ is converted into aglycone form. As a result of the treatment carried out as shown in Table 2, a large quantity of isoflavone compounds of the soybean germ which contains daidzein aglycone as the primary component are obtained.

**Table 2 Units: mg/100 mg**

| Component | | The present invention | Untreated |
|---|---|---|---|
| Glycoside | Daidzin | 70 | 840 |
| | Genistin | 40 | 170 |
| | Glycitin | 130 | 620 |
| | Total | 240 | 1630 |
| Aglycone | Daidzein | 680 | 10 |
| | Genistein | 130 | 2.4 |
| | Glycitein | 240 | 2.0 |
| | Total | 1050 | 14.4 |

The koji mold used in the above koji preparation may be koji mold that is traditionally used in tempeh or traditional Japanese fermented foodstuffs. Examples include koji mold comprising *Aspergillus usami, Aspergillus kawachi, Aspergillus awamori, Aspergillus saitoi, Aspergillus oryzae, Aspergillus niger* and other *Aspergilli* or *Rhizopus* which are safe for use as foodstuffs.

The fermenting time depends on the type of koji mold that is used and is at least 24 hours, but a koji preparation time should be used that is sufficient to provide adequate decomposition of the isoflavone compound glycosides present in soybean meal.

Regarding protein hydrolysis, it is desirable for the hydrolysis temperature and hydrolysis time to be sufficient to allow adequate reduction in isoflavone compound glycoside amounts present in the soybean germ in accordance with the type of koji mold that is used.

By the means above, organic acid is generated during the initial period of koji preparation, and contaminant microorganism propagation in the soybean germ is inhibited, thereby eliminating worries concerning secondary contamination. As a result, mass production of product that uses soybean germ as a raw material is possible. In addition, a treatment can be carried out whereby the glycoside content in the isoflavone compounds is sufficiently reduced without reduced water content.

Next, in this embodiment, in accordance with the method presented in Japanese Patent Application Laid-Open (Kokai) No. 2000-281673, with the use of the above resultant product obtained by hydrolysis (1) solvent extraction is performed using hydrophilic organic solvent, (2) liquid/liquid separation is performed using hydrophobic organic solvent on the hydrophilic organic solvent obtained by extraction, (3) liquid/liquid separation is performed using hydrophobic organic solvent on the hydrophilic organic solvent obtained by liquid/liquid separation, and (4) the hydrophobic organic solvent obtained by liquid/liquid extraction is subjected to concentration and drying as necessary. As a result, AglyMax which is a concentrate having an isoflavone aglycone content of 30 wt% or greater as shown in Table 3 is obtained. The daidzein content in this case is about 70 wt% of the isoflavone aglycone. Table 3 shows the ratios of diadzein, glycitein, and genistein to the amount of isoflavone aglycone. The superoxide scavenging activity of this AglyMax is 160,000 units/g.

**Table 3 Composition of Concentrate of Material of the Present Invention**

| Analytical parameters | Standard value | Analytical value | Content ratio |
|---|---|---|---|
| Condition | Light brown powder, no abnormal flavor, odor or impurities | Passed | |
| Drying weight loss | 5% or less | 2.8% | |
| Combustion residue | 3% or less | 0.1% | |
| Arsenic | 2 ppm or less | Passed | |
| Heavy metals | 20 ppm or less | Passed | |
| Residual agrochemicals | Not detected | Not detected | |
| Viable cell count | 3,000/g or less | 3,000 /g or less | |
| Coliform | Negative | Negative | |
| Isoflavone aglycone content | 30% or greater | 32.9% | 1 |
| Daidzein | | 23.4% | 0.72 |
| Glycitein | | 8.0% | 0.24 |
| Genistein | | 1.5% | 0.04 |

### Examples

The action of the pharmaceutical composition for treating sudden deafness containing isoflavone aglycone derived from soybean germ of the present invention will now be described.

### Example 1

In this Example, a subject with sudden deafness (female in her thirties) was administered a supplement of AglyMax in tablet form such that she ingested 40 mg per day of isoflavone aglycone. The study period was three months. The state of improvement in symptoms before and after administration was confirmed by the survey shown in Table 4. Improvement in hearing was also tested with an audiometer.

As a result, the sudden hearing loss improved over the course of the study. Essentially, the symptom of vertigo was greatly alleviated. Hearing was markedly improved.

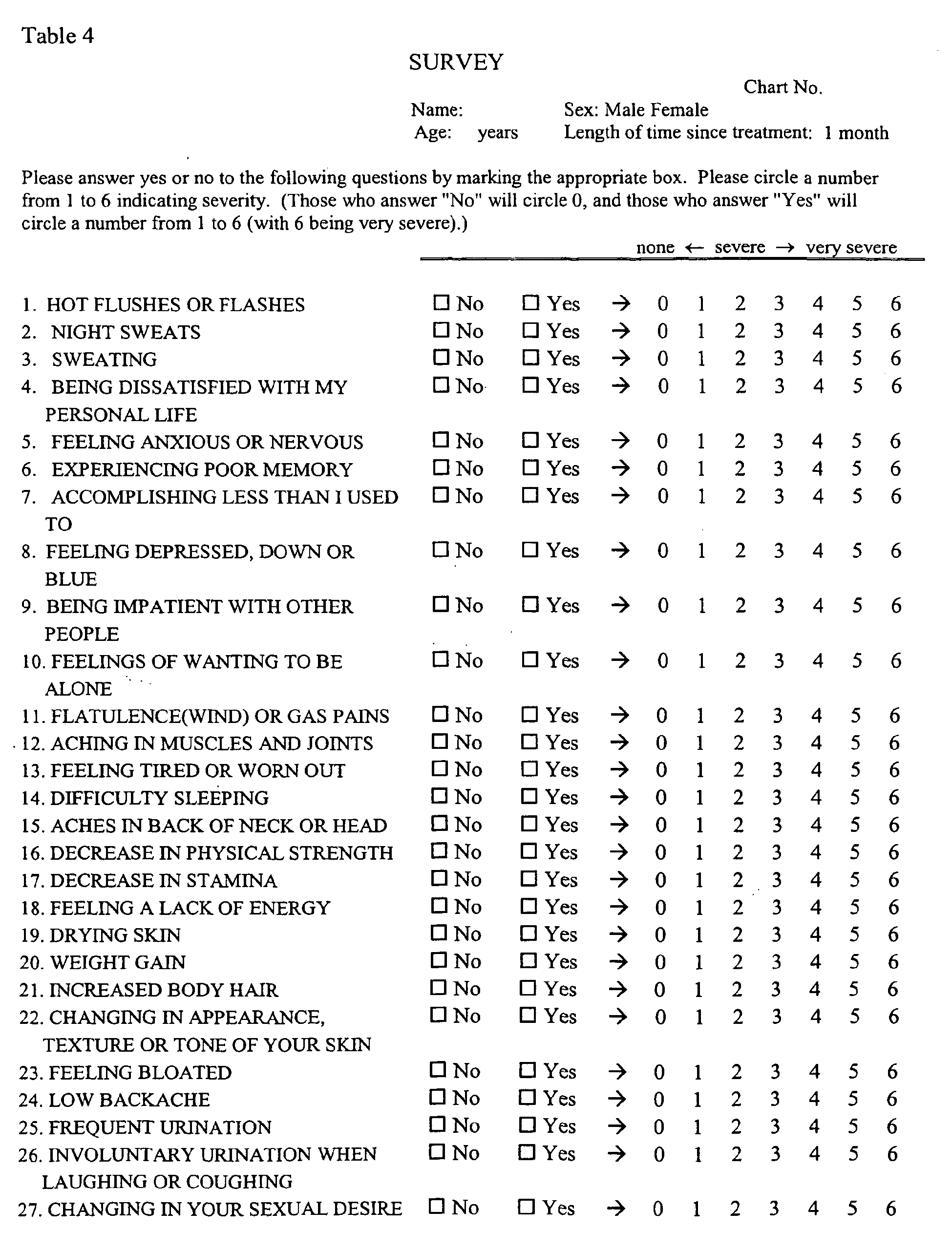

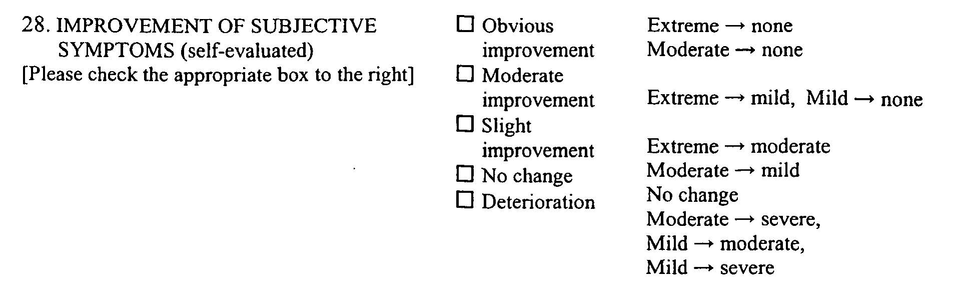

### Example 2

In this Example, subjects (seven men and 19 women; average age of 54.9 ± 13.8 years (men: 54.7 ± 13.7 (27 to 70) years, women: 55.9 ± 15.2 (36 to 79) years) with sudden deafness who did not recover their hearing even when steroid drops were used were administered a supplement of AglyMax in tablet form such that they ingested 40 mg per day of isoflavone aglycone. The test period was three months. Tinnitus and vertigo were assessed by evaluation of the QOL before and after administration through interviews conducted by a physician based on the survey shown in Table 4. Improvement in hearing was also tested with an audiometer. Changes in blood chemistry of the subjects were also investigated.

### Results

### 1) Recovery of hearing

Number recovering 5 dB: 7/26 (26.9%)
Number recovering 2 dB: 6/26 (23.1 %)
Number not recovering: 13/26 (50.0%)

Half of the subjects showed improved hearing, and approximately half of those showing improvement recovered by 5 dB, with hearing loss being completely cured.

### 2) Recovery from vertigo

Number of subjects noticing vertigo: 13/26 (50%)
Number completely recovering: 10/13 (76.9%)
Number partially recovering: 3/13 (32.19%)

Improvement of vertigo was seen in all subjects who noticed vertigo, and subjects completely recovering from vertigo reached 76.9%.

### 3) Recovery of tinnitus

Number of patients noticing tinnitus: 19/26 (73.1%)
Number completely recovering: 8/19 (42.1%)
Number partially recovering: 8/19 (42.1%)
Number who did not recover: 3/19 (15.8%)

Improvement of tinnitus was reported in 84.2% of patients who noticed tinnitus, and subjects completely recovering from tinnitus reached 42.1%.

### 4) Recovery of blood lipid metabolism (corresponding to total cholesterol level)

Number of subjects with anomalous level: 13/26 (50.0%)
An anomalous total cholesterol level here is 219 mg/dL or higher.
Number recovering: 9/13 (69.2%)
Number not recovering: 4/13 (30.8%)

Recovery of blood lipid metabolism (corresponding to total cholesterol level) was seen in 69.2% of subjects with anomalous blood lipid metabolism.

### 5) Recovery of blood lipid metabolism (corresponding to triglyceride level)

Number of subjects with anomalous level: 11/26 (42.3%)
An anomalous triglyceride level here is 149 mg/dL or higher.
Number recovering: 7/11 (63.6%)
Number not recovering: 4/11 (36.4%)

Recovery of blood lipid metabolism (corresponding to triglyceride level) was seen in 63.6% of subjects with anomalous blood lipid metabolism.

### 6) Recovery of blood sugar

Number of subjects with anomalous level: 8/26 (30.8%)
An anomalous blood sugar level here is 110 mg/dL or higher.
Number recovering: 5/8 (62.5%)
Number not recovering: 3/8 (37.5%)

Recovery of blood sugar levels was seen in 62.5% of subjects with anomalous blood sugar levels.

### 7) Improvement of QOL

The QOL of 61.2% of the female subjects improved, while the QOL of 16.5% of the female subjects did not improve. Though the other subjects were interviewed, it was not possible to clearly evaluate whether or not there was improvement in their QOL. Thus, the QOL improved in 61.2% of the female subjects.

The result of treating sudden deafness in this Example 2 was that the present invention was more effective in women than in men.

Such excellent therapeutic results as described above were apparently realized through the effect of this AglyMax supplement, and in particular, were the effect of the strong superoxide scavenging activity of AglyMax.

It should be noted that the present invention is not limited to the above-described Embodiments or Examples and the Embodiments and Examples can be changed as needed. In addition to the oral administration as in the above-described Examples, the pharmaceutical composition for treating sudden deafness containing, as the active ingredient, isoflavone aglycone derived from soybean germ of the present invention can also be adsorbed *in vivo* by instillation.

The present invention is not limited to the above-described Embodiments or Examples and can be changed as needed. For instance, the isoflavone aglycone is not limited to that contained in soybean germ; the isoflavone aglycone contained in a part of the soybean other than the germ or another legume or other plant can be produced as in the case of the soybean germ. Furthermore, the pharmaceutical composition of the present invention can contain a variety of isoflavone aglycones produced by chemical synthesis, and the like.

## Claims

1. A pharmaceutical composition for treating sudden deafness containing, as an active ingredient, isoflavone aglycone.

2. A pharmaceutical composition for treating sudden deafness containing, as an active ingredient, a product that is an isoflavone aglycone concentrate and is obtained by fermenting soybean germ, that is a raw material, using koji mold thus decomposing proteins; then executing hydrolysis thereon; and extracting and concentrating a resulting product using a solvent.

3. The pharmaceutical composition for treating sudden deafness according to Claim 2, **characterized in that** a composition of the isoflavone aglycone concentrate contains at least 70 wt% daidzein.

4. The pharmaceutical composition for treating sudden deafness according to Claim 2, **characterized in that** the product that is an isoflavone aglycone concentrate has strong superoxide scavenging activity.
